(19) Europäisches Patentamt
European Patent Office
Office européen des brevets

(11) EP 0 572 227 B1

# (12) EUROPEAN PATENT SPECIFICATION

(45) Date of publication and mention of the grant of the patent:
**23.08.2000 Bulletin 2000/34**

(51) Int. Cl.$^7$: **C08F 2/26**, C09J 133/06, C09J 133/12, A61K 6/00

(21) Application number: **93304064.4**

(22) Date of filing: **26.05.1993**

## (54) Use of an emulsion of a polymer comprising (meth)acrylate units as a dental adhesive

Verwendung einer Emulsion eines Polymers enthaltend (Meth)Acrylat-einheiten als Dentalklebstoff

Utilisation d'une emulsion de polymère ayant des unités (meth)acrylate comme un adhesif dentaire

(84) Designated Contracting States:
**DE FR GB**

(30) Priority: **27.05.1992 JP 15894592**

(43) Date of publication of application:
**01.12.1993 Bulletin 1993/48**

(73) Proprietors:
- **MITSUI CHEMICALS, INC.
Tokyo (JP)**
- **Nakabayashi, Nobuo
Matsudo-shi, Chiba-ken (JP)**

(72) Inventors:
- **Nakabayashi, Nobuo
Matsudo-shi, Chiba-ken (JP)**
- **Ishihara, Kazuhiko
Kodaira-shi, Tokyo (JP)**
- **Saimi, Yasukazu,
c/o Sun Medical Co., Ltd.
Moriyama-shi, Shiga-ken (JP)**

(74) Representative:
**Cresswell, Thomas Anthony et al
J.A. KEMP & CO.
14 South Square
Gray's Inn
London WC1R 5LX (GB)**

(56) References cited:
- **CHEMICAL ABSTRACTS, vol. 116, no. 18, 4 May 1992, Columbus, Ohio, US; abstract no. 181077n,**
- **CHEMICAL ABSTRACTS, vol. 111, no. 8 Columbus, Ohio, US; abstract no. 58884t,**
- **CHEMICAL ABSTRACTS, vol. 97, no. 2 Columbus, Ohio, US; abstract no. 6983x,**
- **CHEMICAL ABSTRACTS, vol. 111, no. 18 Columbus, Ohio, US; abstract no. 154575p,**
- **CHEMICAL ABSTRACTS, vol. 98, no. 20, 16 May 1983, Columbus, Ohio, US; abstract no. 161233s,**
- **CHEMICAL ABSTRACTS, vol. 88, no. 14 Columbus, Ohio, US; abstract no. 90443r,**
- **DATABASE WPI Week 9116, Derwent Publications Ltd., London, GB; AN 91-112723**
- **CHEMICAL ABSTRACTS, vol. 97, no. 10, 6 September 1982, Columbus, Ohio, US; abstract no. 72878t,**
- **PATENT ABSTRACTS OF JAPAN vol. 15, no. 12 (C-0795)10 January 1991**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 572 227 B1

## Description

The present invention relates to use of dental adhesive compositions containing an emulsion of a polymer having a (meth)acrylate unit.

More specifically, it relates to use of a polymer emulsion which reacts to be fixed on the surface of a solid containing a polyvalent cation reactive with a sulfonic acid group, such as a hydroxyapatite structure, cement, a metal compound or a mineral, use of an adhesive film from the polymer emulsion formed on the surface of said solid and use of an adhesive composition which exhibits excellent adhesion to a substrate.

For treating a hydroxyapatite structure such as a dentin for remedy, an adhesive material such as 4-META/MMA-TBB (Superbond, supplied by Sun Medical Co. Ltd) has been hitherto used. The adhesive material is bonded by a method in which a drilling dust is removed by preliminary etching of an adherend surface with an acid, the adhesive material is applied and cured and then a radical-polymerizable acrylic resin is filled and cured. Since, however, the acid used for the above acid treatment is a low molecular weight compound, it may infiltrate deep into the hydroxyapatite structure. Therefore, when a dentin is treated for remedy, the acid comes to remove a healthy part of the dentin as well and sometimes reaches a dental pulp to attack the nerve and give an acute pain.

Therefore, when a portion near to a dental pulp is treated for remedy, there has been conventionally employed a method in which the attack in bonding is prevented by applying onto the above portion a carboxylate cement which is a combination of a polymer type polyacrylic acid with zinc oxide or a glass ionomer cement which Is a combination of a homopolymer of polyacrylic acid or a copolymer from acrylic acid and either itaconic acid or maleic acid with aluminosilicate glass.

Since, however, these cements are very poor in adhesion, there have been problems such as secondary caries and coming off of the attachment for remedy.

For the therapeutical treatment of the hypersensitivity of a dentin based on exposure of dental tubules in the mouth, there are used a liniment of silver nitrate, potassium oxalate or disodium hydrogenphosphate/calcium chloride (Ikemura, Imai, Journal of Japanese Dentistry Preservation Society, vol. 35, 26 (1992)), a toothpaste containing citric acid or sodium citrate, a glass ionomer cement and a 4-META-containing adhesive resin (D liner, supplied by Sun Medical Co., Ltd).

The above preparations against the hypersensitivity of a dentin have the following advantages. The liniment is free of stimulation to a dental pulp and coloring, and the toothpaste obviates the therapeutical treatment in a dentist's chair. However, it is usual that it takes a long period of time before they exhibit their effects. The polymer type carboxylic acid such as glass ionomer hardly affects a dentin. However, it is poor in adhesion to a dentin and resistance to water, and its effect does not continue satisfactorily. On the other hand, the adhesive resin shows its effect, but is used for the therapy by a method in which a portion to be treated is preliminarily etched with a low molecular weight acid such as a 10 % citric acid - 3 % ferric chloride aqueous solution or the like, and a solution prepared by mixing a curing agent and a monomer immediately before use is applied and polymerized. Therefore, it still involves a problem in the influence of the low molecular weight compound on a dentin and handling.

For decreasing the infiltration of an acid into a hydroxyapatite structure and imparting it with affinity to an acrylic resin, the present Applicant has proposed an acrylate copolymer containing a sulfonic acid group which allows to ion-bond or chelate-bond to a calcium component of the hydroxyapatite structure (JP-A-6203309 and JP-A-2261442).

The polymer disclosed in the above Japanese Patent Publications is specifically an acrylic copolymer produced from methacrylate such as methyl methacrylate and a sulfonic acid group-containing monomer such as p-styrenesulfonic acid and/or methacrylate macromer having a vinyl group at its terminal such as methyl polymethacrylate. These Publications disclose a method in which the above acrylic copolymer is dissolved in a solvent such as water or ethanol, the resultant solution is applied directly to a hydroxyapatite structure surface without acid etching and the resultant coating is washed with water. In this method, the degree of infiltration into the hydroxyapatite structure is small, the copolymer firmly bonds to the hydroxyapatite structure since the sulfonic acid group bonds to a calcium component of the hydroxyapatite structure, and the hydroxyapatite structure can be bonded to an acrylic resin to form a layer thereon since the polymethyl methacrylate portion of the acrylic copolymer and the acrylic resin have good affinity.

However, further studies of the above acrylic copolymer have revealed the following. When a solution of the acrylic copolymer is applied and dried without washing it with water, the acrylic copolymer sometimes shows no adhesion force due to a presence of a remaining unreacted acrylic copolymer which does not react with the hydroxyapatite structure. It is therefore essential to carry out a step of washing with water for obtaining a stable adhesion force.

It is an object of the present invention to overcome the above problems.

It is another object of the present invention to provide a composition for use as a dental adhesive which decreases the infiltration into an adherend, like a conventional sulfonic acid group-containing copolymer, which exhibits an adhesion force to a hydroxyapatite structure, a cement, a metal or a mineral when it is applied thereto and, without washing it with water, only dried around room temperature to form a film, which exhibits excellent affinity to a radical-polymerizable acrylic resin composition, and which further permits sealing the surface of a hypersensitive dentin since a film formed therefrom has the property of bonding to a dentin which is a hydroxyapatite structure.

Other objects and advantages of the present invention will be apparent from the following description.

According to the present invention, the above objects and advantages of the present invention are achieved, first, by use of a composition containing an emulsion of a polymer comprising (a) a recurring unit derived from a (meth)acrylate and (b) a recurring unit derived from a vinyl compound having a group of $-SO_3R$ in which R is a hydrogen atom, an alkali metal atom or ammonium ion as a dental adhesive.

The polymer emulsion may be an emulsion of a polymer which may further contain (c) a recurring unit derived from a vinyl compound containing a group of $-COOR_4$ in which $R_4$ is a hydrogen atom, an alkali metal atom or ammonium ion, or a group of $-OPO(OR_5)_2$ in which $R_5$ is a hydrogen atom, an alkali metal atom or ammonium ion, in addition to the above recurring units (a) and (b).

The above (a) recurring unit derived from (meth)acrylate has the formula (A) for example,

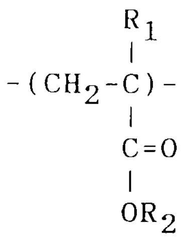

(A)

wherein $R_1$ is a hydrogen atom or an alkyl group having 1 to 5 carbon atoms and $R_2$ is an alkyl group having 1 to 5 carbon atoms.

The above (b) recurring unit derived from a vinyl compound having a group of $-SO_3R$ has the formula (B),

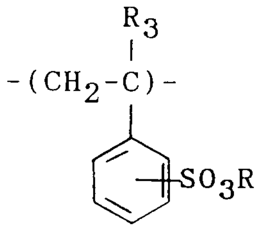

(B)

wherein $R_3$ is a hydrogen atom or an alkyl group having 1 to 5 carbon atoms and R is a hydrogen atom, an alkali metal atom or ammonium ion.

A polymer emulsion may be obtained by emulsion-polymerizing (meth)acrylate in the presence of the above emulsion polymer as an emulsifying agent.

The recurring unit derived from the (meth)acrylate preferably has the above formula (A).

The polymer emulsion is produced, for example, by emulsion-polymerizing a (meth)acrylate of the formula (A1),

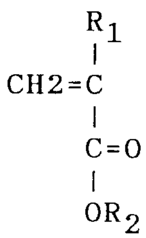

(A1)

wherein $R_1$ and $R_2$ are as defined in the above formula (A), with a sulfonic acid group-containing monomer of the formula (B1),

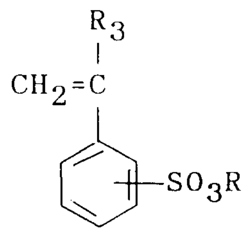

(B1)

wherein $R_3$ and R are as defined in the above formula (B),

preferably in an (A1):(B1) molar ratio of 99:1 to 50:50, or by homopolymerizing one (meth)acrylate monomer of the formula (A1), or copolymerizing (meth)acrylate monomers of the formula (A1), in the presence of the so-obtained emulsion polymer as an emulsifying agent.

Further, in the production of the polymer emulsion, the polymer emulsion is produced by emulsion-polymerizing the vinyl compounds of the above formulae (A1) and (B1) and either (E) a vinyl compound having a group of $-COOR_4$ or (F) a vinyl compound having a group of $-OPO(OR_5)_2$ in which $R_4$ and $R_5$ are as defined above, preferably in an (A1):(B1)+(E) or (A1):(B1)+(F) molar ratio of 99:1 to 50:50 and in a (B1):(E) or (B1):(F) molar ratio of 99:1 to 1:99, or by homopolymerizing one (meth)acrylate monomer of the formula (A1), or copolymerizing (meth)acrylate monomers of the formula (A1), in the presence of the so-obtained emulsion polymer as an emulsifying agent.

Being constituted by the specific recurring units, the emulsion polymer obtained can form a film having the excellent property of bonding to a sulfonic acid group of a hydroxyapatite structure, a cement, a metal compound or a mineral. Therefore, a dentin such as a hypersensitive dentin surface can be sealed with an adhesive film. Further, since it shows excellent affinity to a radical-polymerizable acrylic resin, it can be properly used as an adhesive for bonding a dentin and a radical-polymerizable acrylic resin.

The adhesive emulsion, process for the production thereof and adhesive composition containing the same will be explained hereinafter.

First, the process for the production of the adhesive emulsion will be explained. In the present invention, the term "emulsion polymer" refers to a polymer obtained by emulsion polymerization, and the term "polymer emulsion" refers to an emulsion of the emulsion polymer.

The polymer emulsion (to be referred to as "EM" hereinafter) can be produced by subjecting a (meth)acrylate of the above formula (A1) and a vinyl compound having a group of $-SO_3R$ in which R is a hydrogen atom, an alkali metal atom or ammonium ion, represented by the above formula (B1), to a known emulsion polymerization method (e.g., "Polymer Latex", Soichi Muroi, Ikuo Morino, published as Shin-Kobunshi Bunko).

In the formula (A1), $R_1$ is a hydrogen atom or an alkyl group having 1 to 5 carbon atoms. Specific examples of the alkyl group include methyl, ethyl, propyl, butyl and pentyl. $R_1$ is preferably an alkyl group having 1 to 5 carbon atoms, more preferably methyl.

Further, $R_2$ is an alkyl group having 1 to 5 carbon atoms. Specific examples of the alkyl group include methyl, ethyl, propyl, butyl and pentyl. $R_2$ is preferably methyl, ethyl or butyl, more preferably methyl.

Examples of the compound of the above formula (A1) preferably include (meth)acrylates such as methyl (meth)acrylate, ethyl (meth)acrylate, butyl (meth)acrylate, propyl (meth)acrylate, pentyl (meth)acrylate, glycidyl (meth)acrylate, 2-hydroxy (meth)acrylate and 3-hydroxy (meth)acrylate.

Of the above (meth)acrylates, preferred are methyl methacrylate, ethyl methacrylate and butyl methacrylate, and particularly preferred is methyl methacrylate.

In the present invention, there is used another monomer having a group of $-SO_3R$ in which R is a hydrogen atom, an alkali metal atom or ammonium ion. Specifically, the monomer includes vinyl compounds or alkyl group-substituted vinyl compounds to which the group of $-SO_3R$ in which R is as defined above bonds directly or through other group.

Examples of the above sulfonic acid group-containing monomer preferably include allylsulfonic acid, methallylsulfonic acid, vinylsulfonic acid, o-styrenesulfonic acid, m-styrenesulfonic acid, p-styrenesulfonic acid, tert-butylacrylamidesulfonic acid, alkali metals salts of these acids such as lithium salts, potassium salts and sodium salts, and ammonium salts of the above acids. Of these, preferred are compounds in which the above group of $-SO_3R$ bonds to a carbon atom which is part of a polymerizable group, such as styrenesulfonic acids. These compounds are preferably represented by the above formula (B1).

In the above formula (B1), $R_3$ is a hydrogen atom or an alkyl group having 1 to 5 carbon atoms. The alkyl group includes methyl, ethyl, propyl, butyl and pentyl. $R_3$ is preferably a hydrogen atom.

The position at which the group of $-SO_3R$ bonds may be any one of the o-position, m-position and p-position in the benzene ring. In view of the adhesion to an adherend, preferred is the p-position. Further, the group of $-SO_3R$

may be any one of a lithium salt, potassium salt, sodium salt and ammonium salt. Examples of the compound of the above formula (B1) preferably include sulfonic acid compounds such as allysulfonic acid, methallylsulfonic acid, vinylsulfonic acid, sulfoethyl methacrylate, tert-butylacrylamidesulfonic acid and p-styrenesulfonic acid, and sodium salts and ammonium salts of these. Of these examples, preferred is p-styrenesulfonic acid.

Examples of the above (E) monomer having a group of -$COOR_4$ in which $R_4$ is a hydrogen atom, an alkali metal atom or ammonium ion include unsaturated monovalent carboxylic acids such as acrylic acid, methacrylic acid, vinylacetic acid, crotonic acid, methacryloyloxybenzoic acid, vinylsalicylic acid and vinylacetylsalicyclic acid; alkali metal salts of these acids; ammonium salt compounds of these acids; unsaturated polyvalent carboxylic acids such as maleic acid, fumaric acid, itaconic acid, aconitic acid, citraconic acid, mesaconic acid and 4-methacryloyloxyethyltrimellitic acid; alkali metal salts of these acids; and ammonium acid compounds of these acids. Of these, preferred are acrylic acid and methacrylic acid.

Examples of the above (F) monomer having a group of -$OPO(OR_5)_2$ in which $R_5$ is a hydrogen atom, an alkali metal atom or ammonium ion include phosphate ester monomers such as acid phosphoethyl (meth)acrylate, 3-chloro-2-acid phosphopropyl (meth)acrylate, acid phosphooxypropyl (meth)acrylate, acid phosphooxypolyoxyethylethylene glycol mono(meth)acrylate and acid phosphooxypolyoxypropylene glycol mono(meth)acrylate; alkali metal salts of these; ammonium salt-containing monomers of these; and those compounds derived from the above compounds in which the hydroxyl group is substituted with other substituent, i.e., phosphate ester type monomers such as 2-(meth)acryloyloxyethylphenyl phosphate and 2-(meth)acryloyloxyethyl 4-methoxyphenyl phosphate. Of these, acid phosphooxyethyl methacrylate or acid phosphooxyethylene glycol monomethacrylate is useful.

In the emulsion polymerization, the molar ratio of the monomor of the above formula (A1) to the monomer of the above formula (B1) is preferably as shown below, since an excellent emulsion can be obtained and since the acidity decreases to prevent the infiltration into an adherend. That is, the molar ratio of the monomer of the formula (A1) and the monomer of the formula (B1) is preferably 99:1 to 50:50, more preferably 95:5 to 60:40. Further, concerning the molar ratio of the monomer of the formula (A1) and the monomer of the formula (B1) and either the (E) vinyl compound having a group of -$COOR_4$ or the (F) vinyl compound having a group of -$OPO(OR_5)_2$ in which $R_4$ and $R_5$ are as defined above, preferably, the (A1):(B1)+(E) or (B1)+(F) molar ratio is 99:1 to 50:50 and that the (B1):(E) or (F) molar ratio is 99:1 to 1:99, and more preferably, the (A1):(B1)+(E) or (B1)+(F) molar ratio is 95:5 to 60:40 and that the (B1):(E) or (F) molar ratio is 99:1 to 5:95.

The reaction conditions for the emulsion polymerization are not specially limited. For example, an excellent polymer emulsion can be produced, for example, by adding a mixture of (A1) and (B1) or (A1), (B1) and optionally either (E) or (F) to water in an amount of 60 parts by weight or less per 100 parts by weight of the water at a temperature ranging from room temperature to 100°C, adding an initiator for emulsion polymerization and polymerizing them for tens minutes to 24 hours. When the monomer mixture is added at one time, an emulsion containing a large amount of aggregates is liable to be formed. It is therefore preferred to add the monomer mixture intermittently.

The emulsion polymerization may be carried out in the presence or absence of a surfactant such as an anionic surfactant, a cationic surfactant, a nonionic surfactant or a polymer surfactant such as polyethylene glycol. In the absence of a surfactant, however, there can be produced a polymer emulsion which is free of aggregates, has high stability in water and has an emulsion particle diameter of 1 μm or less. For simple post-treatment and for avoiding the impairment of the adhesion and film strength of the polymer emulsion, it is preferred to carry out soap-free emulsion polymerization in the absence of a surfactant. Further, when the above monomer (E) or (F) is used, it is preferred to produce the emulsion under an acidic condition of pH 6 or less for maintaining the emulsion stability in a good state.

In addition to the above monomers, other monomer which is generally emulsion-polymerizable may be copolymerized in such an amount that does not impair the properties of the adhesive composition of the present invention. Examples of the "other monomer" include glycidyl esters such as N-(2-hydroxy-3-methacryloyloxypropyl)-N-phenylglycine, glycidyl (meth)acrylate and N-acrylglycine; (meth)acrylates having an alkylamino group such as N,N-dimethylaminoethyl (meth)acrylate, aminoethyl (meth)acrylate and hydroxyethylaminoethyl (meth)acrylate; olefins such as ethylene, propylene and 1-butene; vinyl halides such as vinyl chloride, vinylidene chloride, vinyl bromide, 2-chloroethyl (meth)acrylate, 1,1-dichloroethylene and tetrachloroethylene; vinyl esters such as vinyl acetate and vinyl propionate; (meth)acrylaldehydes such as (meth)acrylaldehyde and 3-cyano(meth)acrylaldehyde; vinyl ethers such as methyl vinyl ether, isobutyl vinyl ether, (meth)acrylaldehyde diacetate, (meth)acrylaldehyde diethyl acetal and 1,2-dimethoxyethylene; alkenylbenzenes such as styrene, vinyltoluene, α-methylstyrene, chloromethylstyrene, stilbene and 1,1-diphenylethylene; vinyl cyanide compounds such as acrylonitrile and methacrylonitrile; (meth)acrylamides such as (meth)acrylamide, N-vinylphthalamide, N-vinylsuccinamide, N,N-dimethylacrylamide, N-hydroxymethyl(meth)acrylamide and N-hydroxyethyl-2-methylacrylamide; and (meth)acrylates having a hydroxyl group such as hydroxylmethyl (meth)acrylate, 2-hydroxyethyl (meth)acrylate and 4-hydroxybutyl (meth)acrylate.

The process for the production of an emulsion polymer by homopolymerizing or copolymerizing (meth)acrylate(s) (A1) in the presence of the above-obtained polymer emulsion (EM) as an emulsifying agent will be explained hereinafter.

The (meth)acrylate(s) used in the above process are the compound(s) of the formula (A1).

In this process, specific examples of the alkyl group as $R_1$ and $R_2$ in the formula (A1) also include methyl, ethyl, propyl, butyl and pentyl. $R_1$ is preferably an alkyl group, more preferably methyl. $R_2$ is preferably methyl.

In this process, preferred examples of the compound of the formula (A1) include alkyl esters of (meth)acrylic acids such as methyl (meth)acrylate, ethyl (meth)acrylate, butyl (meth)acrylate, propyl (meth)acrylate, pentyl (meth)acrylate and glycidyl (meth)acrylate.

Of the above (meth)acrylates, methyl methacrylate is particularly preferred.

The method of emulsion-polymerization of the (meth)acrylate of the formula (A1) has a characteristic feature in that the above-detailed polymer emulsion "EM" is used as an emulsifying agent.

The above polymer emulsion "EM" may be added to the (meth)acrylate compound of the formula (A1) as an emulsifying agent after it has been taken out of a reaction vessel. Further, the above polymer emulsion "EM" may be used in a manner in which the (meth)acrylate compound of the formula (A1) is added to it while it is in a reaction vessel, i.e., without being taken out of a reaction vessel, after it has been produced.

The reaction conditions are not specially limited, while, for example, the polymer emulsion can be produced by adding the (meth)acrylate compound of the formula (A1) to the polymer emulsion "EM" within the temperature range between room temperature and 100°C after the polymer emulsion has been produced, and emulsion-polymerizing the resultant mixture for tens minutes to 24 hours.

The mixing ratio of the polymer emulsion "EM" and the monomer of the formula (A1) and the amounts of these based on water are not specially limited. Preferably, the "EM":(A1) weight ratio is 1:99 to 80:20, and the total amount of "EM" and the monomer (C) per 100 parts by weight of water is 60 parts by weight or less.

The type of the emulsion polymer and the composition of the copolymer can be varied by intermittently changing the kind of the (meth)acrylate compound (A1) and the composition of the mixture in the polymerization system.

In the above polymer emulsion, other monomer may be compolymerized in addition to the compound of the formula (A1) in such an amount that does not impair the adhesion ability and film strength of the polymer emulsion of the present invention. The above "other monomer" includes generally emulsion-polymerizable monomers. That is, the "other monomer" is selected from olefins such as ethylene, propylene and butene-1; vinyl halides such as vinyl chloride, vinylidene chloride and vinyl bromide; vinyl esters such as vinyl acetate and vinyl propinonate; vinyl ethers such as methyl vinyl ether, ethyl vinyl ether and isobutyl vinyl ether; alkenylbenzenes such as styrene, vinyltoluene, α-methylstyrene, chloromethylstyrene and stilbene; and vinyl cyanide compounds such as acrylonitrile and methacrylonitrile.

The so-produced polymer emulsion exhibits excellent stability in water for a long period of time and can be also formed into a film which exhibits excellent adhesion to an adherend structure having a polyvalent cation reactive to a sulfonic acid group, since the emulsion polymer has a sulfonic acid unit.

When the polymer emulsion is used as an adhesive or a surface treating agent, it is preferred to use it in a state in which it is dispersed in a polar solvent such as water and an alcohol, e.g. as ethanol. In particular, when it is used for a dental purpose, it is preferred to use it in a dispersion in water or a water-ethanol mixed solvent. The solid content of the polymer emulsion in the above dispersing medium is generally 0.05 to 20 % by weight, preferably 0.5 to 15 % by weight.

For improving the reactivity of the above polymer emulsion with an adherend, a low molecular weight acid may be optionally added in such an amount that does not impair the stability of the emulsion and the water resistance of the film, i.e., in an amount of several to thousands in terms of ppm unit. The low molecular weight acid includes citric acid, oxalic acid, maleic acid, phosphoric acid and EDTA.

The above polymer emulsion may contain known additives as required, such as a coloring pigment, a loading pigment, an aggregate, a wetting agent and a thickener.

A film can be formed simply by applying the polymer emulsion to an adherend having a polyvalent cation reactive with a sulfonic acid group, allowing it to stand for a few seconds to a few minutes and drying it with compressed air or the like at room temperature. It is assumed that the sulfonic acid group of each emulsion particle being oriented in the solvent direction chemically bonds to an adherend during the above process, whereby each emulsion particle bonds to the adherend.

Further, the dried film is completely removed from an adherend unreactive with a sulfonic acid group such as glass by washing it with water, while it is maintained on an adherend reactive with a sulfonic acid group such as hydroxyapatite even when it is washed with water. Therefore, formation of a film firmly bonded to a dentin can be attained, and it is suitable as a dentin protection film such as a sealing film against the hypersensitivity of a dentin. Further, this film can serve to bond an adherend to an acrylic resin, since it contains a poly(meth)acrylate component having high affinity to the acrylic resin.

The polymer emulsion is, as described above, an emulsion of a copolymer containing recurring units of a sulfonic acid group and a (meth)acrylate, or an emulsion of a copolymer containing recurring units of a sulfonic acid group, a (meth)acrylate and either a carboxyl group or a phosphate ester group. Therefore, it can be formed into a film

which exhibits adhesion to an adherend containing a polyvalent cation reactive with a sulfonic acid group. Further, it also exhibits excellent affinity to a radical-polymerizable acrylic resin. Since the formed film is firmly bonded to an adherend surface which can react with a sulfonic acid group, it is not removed by washing it with water. This film is useful as a dentin protection film, particularly useful against the hypersensitivity of a dentin. As will be described in Example 44 later, this film has been found to have an effect on at least 90 % of volunteer patients, thus giving an excellent clinical result. When an attempt has been made to bond a hydroxyapatite structure to a material for remedy by using the polymer emulsion, it has shown a maximum adhesion strength of 8,2 MPa, while commercially available materials (Comparative Examples 5 to 8) and linear (meth)acrylate/p-styrenesulfonic acid copolymers (without the procedures of washing with water) (Comparative Examples 9 and 10) have shown an adhesion strength of about 0 to 0.3 MPa. With the polymer emulsion, the therapeutical treatment of the hypersensitivity of a dentin and the operation of a hydroxyapatite structure for remedy can be performed with simple procedures and with more reliability.

The present invention will be explained hereinafter more in detail by reference to Examples, which, however, shall not be limitative to the present invention.

In the Examples, methyl methacrylate and acrylic acid were distilled under reduced pressure, and then dissolved oxygen was removed by degassing under nitrogen bubbling just before use. Acid phosphooxyethyl methacrylate (Phosmer M, supplied by Unichemical Co., Ltd.) and sodium p-styrenesulfonate (Spinomer, purity 85 %, supplied by Tosoh Corp.) were used as they were commercially available.

Example 1 (E-1)

Distilled water (25 ml) was temperature-increased to 60° C, and a nitrogen gas was bubbled therein for 1 hour. Under nitrogen atmosphere, 1.0 g of methyl methacrylate (hereinafter referred to as "MMA"), 0.27 g of sodium p-styrenesulfonate (hereinafter referred to as "SSNa", Spinomer, supplied by Tosoh Corp.), and 30 mg of potassium persulfate and 10 mg of sodium sulfite as polymerization initiators were added, and the mixture was vigorously stirred at 60° C for 2 hours. 4.0 Grams of MMA, 1.1 g of SSNa, 70 mg of potassium persulfate and 20 mg of sodium phosphite were further added, and the mixture was vigorously stirred for 22 hours and then cooled to room temperature to give an emulsion having a solid content of 5.8 wt.%. Concentrated hydrochloric acid (0.47 ml) was added, and the mixture was stirred for 2 hours and then placed in a dialysis tube. The mixture was dialyzed in distilled water for 5 days while the distilled water was changed to new one each day. The dialysis tube was dried at room temperature under atmospheric pressure to give an emulsion having a solid content of 8.5 wt.%.

The infrared spectrum (IR) of the above-obtained emulsion polymer showed that it contained MMA and styrenesulfonic acid units. When the polymer was also analyzed with GPC using, as a reference, polymethyl methacrylate whose molecular weight was known, it had a number average molecular weight ($\overline{Mn}$) of 1.0 x $10^6$. Further, the elemental analysis of the polymer showed that the MMA unit content was 91.0 mol%.

Example 2 (E-2)

Distilled water (32 ml) was temperature-increased to 60° C, and a nitrogen gas was bubbled therein for 1 hour. Under nitrogen atmosphere, 1.0 g of MMA, 0.16 g of SSNa, 30 mg of potassium persulfate and 10 mg of sodium sulfite were added, and the mixture was vigorously stirred at 60° C for 2 hours. Then, 1 g of MMA, 0.16 g of SSNa, 10 mg of potassium persulfate and 3 mg of sodium sulfite were intermittently added six times at intervals of 30 minutes with the same components and amounts as described above each time, and the mixture was vigorously stirred for 19 hours. The reaction mixture was cooled to room temperature, and after 0.38 ml of concentrated hydrochloric acid was added, the mixture was fully stirred for 2 hours. The mixture was placed in a dialysis tube and dialyzed in distilled water for 5 days while the distilled water was changed to new one each day. The dialysis tube was dried at room temperature under atmospheric pressure to give an emulsion having a solid content of 10.7 wt.%.

The IR of the above emulsion polymer showed that it contained MMA and styrenesulfonic acid units. The polymer was analyzed in the same manner as in Example 1 to show $\overline{Mn}$ of 7.5 x $10^5$. Further, the elemental analysis of the polymer showed that the MMA unit content was 94.1 mol%.

Example 3 (E-3)

Distilled water (35 ml) was temperature-increased to 60° C, and a nitrogen gas was bubbled therein for 1 hour. Under nitrogen atmosphere, 1.0 g of MMA, 0.12 g of SSNa, 30 mg of potassium persulfate and 10 mg of sodium sulfite were added, and the mixture was vigorously stirred at 60° C for 2 hours. Then, 2.0 g of MMA, 0.24 g of SSNa, 30 mg of potassium persulfate and 10 mg of sodium sulfite were added, and the mixture was vigorously stirred for 30 minutes. Then, 4.0 g of MMA, 0.48 g of SSNa, 60 mg of potassium persulfate and 20 mg of sodium sulfite were added, and the mixture was vigorously stirred for 21.5 hours. The reaction mixture was cooled to room temperature, and after

0.29 ml of concentrated hydrochloric acid was added, the mixture was stirred for 2 hours. The mixture was placed in a dialysis tube and dialyzed in distilled water for 5 days while the distilled water was changed to new one each day. The dialysis tube was dried at room temperature under atmospheric pressure to give an emulsion having a solid content of 11.2 wt.%.

The IR of the above emulsion polymer showed that it contained MMA and styrenesulfonic acid units. The polymer was analyzed in the same manner as in Example 1 to show $\overline{Mn}$ of 6.2 x $10^5$. Further, the elemental analysis of the polymer showed that the MMA unit content was 95.7 mol%.

Example 4 (E-4)

Distilled water (41 ml) was temperature-increased to 60° C, and a nitrogen gas was bubbled therein for 1 hour. Under nitrogen atmosphere, 1.0 g of MMA, 0.08 g of SSNa, 30 mg of potassium persulfate and 10 mg of sodium sulfite were added, and the mixture was vigorously stirred at 60° C for 2 hours. Further, 2.0 g of MMA, 0.16 g of SSNa, 23 mg of potassium persulfate and 3 mg of sodium sulfite were added three times at intervals of 30 minutes, and further, the mixture was vigorously stirred for 21.5 hours. The reaction mixture was cooled to room temperature, and after 0.19 ml of concentrated hydrochloric acid was added, the mixture was stirred for 2 hours. The mixture was placed in a dialysis tube and dialyzed in distilled water for 5 days while the distilled water was changed to new one each day. The dialysis tube was dried at room temperature under atmospheric pressure to give an emulsion having a solid content of 16.9 wt.%.

The IR of the above emulsion polymer showed that it contained MMA and styrenesulfonic acid units. The polymer was analyzed in the same manner as in Example 1 to show $\overline{Mn}$ of 5.9 x $10^5$. Further, the elemental analysis of the polymer showed that the MMA unit content was 96.7 mol%.

Example 5 (E-5)

Distilled water (55 ml) was temperature-increased to 60° C, and a nitrogen gas was bubbled therein for 1 hour. Under nitrogen atmosphere, 1.0 g of MMA, 0.06 g of SSNa, 30 mg of potassium persulfate and 10 mg of sodium sulfite were added, and the mixture was vigorously stirred at 60° C for 2 hours. Further, 2.0 g of MMA, 0.12 g of SSNa, 23 mg of potassium persulfate and 3 mg of sodium sulfite were added three times at intervals of 30 minutes, and further, the mixture was vigorously stirred for 21.5 hours. The resultant emulsion had a solid content of 8.8 wt.%. The emulsion was cooled to room temperature, and after 0.14 ml of concentrated hydrochloric acid was added, the mixture was stirred for 2 hours. The mixture was placed in a dialysis tube and dialyzed in distilled water for 5 days while the distilled water was changed to new one each day. The dialysis tube was dried at room temperature under atmospheric pressure to give an emulsion having a solid content of 14.7 wt.%.

The IR of the above emulsion polymer showed that it contained MMA and styrenesulfonic acid units. The polymer was analyzed in the same manner as in Example 1 to show Mn of 1.6 x $10^6$. Further, the elemental analysis of the polymer showed that the MMA unit content was 97.6 mol%.

Example 6 (E-6)

Distilled water (50 ml) was temperature-increased to 60° C, and a nitrogen gas was bubbled therein for 1 hour. Under nitrogen atmosphere, 2.0 g of MMA, 1.2 g of SSNa, 30 mg of potassium persulfate and 10 mg of sodium sulfite were added, and the mixture was vigorously stirred at 60° C for 2.5 hours. Further, 1.0 g of MMA, 15 mg of potassium persulfate and 7 mg of sodium sulfite were added four times at intervals of 30 minutes, and further, the mixture was vigorously stirred for 19.5 hours. The reaction mixture was cooled to room temperature, and after 0.4 ml of concentrated hydrochloric acid was added, the mixture was stirred for 2 hours. The mixture was placed in a dialysis tube and dialyzed in distilled water for 5 days while the distilled water was changed to new one each day. The dialysis tube was dried at room temperature under atmospheric pressure to give an emulsion having a solid content of 9.1 wt.%.

The elemental analysis of the polymer showed that the MMA unit content was 92.6 mol%.

Example 7 (E-7)

Distilled water (50 ml) was temperature-increased to 60° C, and a nitrogen gas was bubbled therein for 1 hour. Under nitrogen atmosphere, 2.0 g of MMA, 0.54 g of SSNa, 30 mg of potassium persulfate and 10 mg of sodium sulfite were added, and the mixture was vigorously stirred at 60° C for 2.5 hours. Further, 1.0 g of MMA, 15 mg of potassium persulfate and 7 mg of sodium sulfite were added four times at intervals of 30 minutes, and further, the mixture was vigorously stirred for 19.5 hours. The reaction mixture was cooled to room temperature, and after 0.19 ml of concentrated hydrochloric acid was added, the mixture was stirred for 2 hours. The mixture was placed in a dialysis tube

and dialyzed in distilled water for 5 days while the distilled water was changed to new one each day. The dialysis tube was dried at room temperature under atmospheric pressure to give an emulsion having a solid content of 10.9 wt.%.

The elemental analysis of the polymer showed that the MMA unit content was 96.9 mol%. This polymer emulsion was observed through a transmission microscope to show that it contains particles having a diameter of 0.1 to 0.5 μm.

Example 8 (E-8)

Distilled water (50 ml) was temperature-increased to 60°C, and a nitrogen gas was bubbled therein for 1 hour. Under nitrogen atmosphere, 2.0 g of MMA, 0.48 g of SSNa, 30 mg of potassium persulfate and 10 mg of sodium sulfite were added, and the mixture was vigorously stirred at 60° C for 2 hours. Further, 1.0 g of MMA, 15 mg of potassium persulfate and 7 mg of sodium sulfite were added four times at intervals of 30 minutes, and further, the mixture was vigorously stirred for 20 hours. The reaction mixture was cooled to room temperature, and after 0.17 ml of concentrated hydrochloric acid was added, the mixture was stirred for 2 hours. The mixture was placed in a dialysis tube and dialyzed in distilled water for 5 days while the distilled water was changed to new one each day. The dialysis tube was dried at room temperature under atmospheric pressure to give an emulsion having a solid content of 10.5 wt.%.

The elemental analysis of the polymer showed that the MMA unit content was 98.5 mol%.

Example 9 (EA-1)

While 60 ml of a hydrochloric acid aqueous solution having pH of 2 was temperature-Increased to 60° C, an argon gas was bubbled therein for 20 minutes. Under argon atmosphere, 2.00 g (20 mmol) of MMA, 0.24 g (3.3 mmol) of acrylic acid (hereinafter referred to as "AA"), 0.61 g (2.5 mmol) of SSNa, 30 mg of potassium persulfate (hereinafter referred to as "KPS") and 10 mg of sodium sulfite (hereinafter referred to as "NHS") were added, and the mixture was vigorously stirred at 60° C for 2.5 hours. 15 Minutes after the monomers had been added, the mixture became a bluish white solution.

Further, 1.00 g (10 mmol) of MMA, 30 mg of KPS and 15 mg of NHS were added, and then 1 g of MMA was added three times at intervals of 20 minutes. After 30 mg of KPS and 15 mg of NHS had been added, the mixture was stirred until the total reaction time was as long as 24 hours. The reaction mixture was cooled to room temperature, and after 0.21 ml of concentrated hydrochloric acid was added, the mixture was further stirred for 2 hours. The mixture was placed in a dialysis tube and dialyzed in distilled water for 7 days while the distilled water was changed to new one each day to give an emulsion having a solid content of 5.7 wt.%.

The IR of the above emulsion polymer showed that it contained MMA, AA and styrenesulfonic acid (hereinafter referred to as "SSA") units. Further, the elemental analysis (hereinafter referred to as "EA") of the polymer showed that the SSA and AA unit contents were 7.2 and 13.1 mol%, respectively.

Example 10 (EA-2)

While 70 ml of a hydrochloric acid aqueous solution having pH of 2 was temperature-increased to 60° C, an argon gas was bubbled therein for 20 minutes. Under argon atmosphere, 2.00 g (20 mmol) of MMA, 0.24 g (3.3 mmol) of AA, 0.29 g (1.2 mmol) of SSNa, 30 mg of KPS and 10 mg of NHS were added, and the mixture was vigorously stirred for 2.5 hours. 15 Minutes after the monomers had been added, the mixture became a bluish white solution.

Further, 1.00 g (10 mmol) of MMA, 30 mg of KPS and 15 mg of NHS were added, and then 1 g of MMA was added three times at intervals of 20 minutes. After 30 mg of KPS and 15 mg of NHS had been added, the mixture was stirred until the total reaction time was as long as 24 hours. The reaction mixture was cooled to room temperature, and after 0.10 ml of concentrated hydrochloric acid was added, the mixture was further stirred for 2 hours. The mixture was placed in a dialysis tube and dialyzed in distilled water for 7 days while the distilled water was changed to new one each day to give an emulsion having a solid content of 8.1 wt.%.

The IR of the above emulsion polymer showed that it contained MMA, SSA and AA units. Further, the EA of the polymer showed that the SSA and AA unit contents were 3.2 and 27.8 mol%, respectively.

Example 11 (EA-3)

While 70 ml of a hydrochloric acid aqueous solution having pH of 2 was temperature-increased to 60° C, an argon gas was bubbled therein for 20 minutes. Under argon atmosphere, 2.00 g (20 mmol) of MMA, 0.24 g (3.3 mmol) of AA, 0.17 g (0.7 mmol) of SSNa, 30 mg of KPS and 10 mg of NHS were added, and the mixture was vigorously stirred for 2.5 hours. 15 Minutes after the monomers had been added, the mixture became a bluish white solution.

Further, 1.00 g (10 mmol) of MMA, 30 mg of KPS and 15 mg of NHS were added, and then 1 g of MMA was

added three times at intervals of 20 minutes. After 30 mg of KPS and 15 mg of NHS had been added, the mixture was stirred until the total reaction time was as long as 24 hours. The reaction mixture was cooled to room temperature, and after 0.06 ml of concentrated hydrochloric acid was added, the mixture was further stirred for 2 hours. The mixture was placed in a dialysis tube and dialyzed in distilled water for 7 days while the distilled water was changed to new one each day to give an emulsion having a solid content of 5.6 wt.%.

The IR of the above emulsion polymer showed that it contained MMA, SSA and AA units.

Example 12 (EP-1)

While 70 ml of distilled water was temperature-increased to 60° C, an argon gas was bubbled therein for 20 minutes. Under argon atmosphere, 2.00 g (20 mmol) of MMA, 0.71 g (3.4 mmol) of Phosmer M, 0.63 g (2.6 mmol) of SSNa, 30 mg of KPS and 10 mg of NHS were added, and the mixture was vigorously stirred for 3 hours. 20 Minutes after the monomers had been added, the mixture became a bluish white solution.

Further, 1.00 g (10 mmol) of MMA, 30 mg of KPS and 15 mg of NHS were added, and then 1 g of MMA was added three times at intervals of 20 minutes. After 30 mg of KPS and 15 mg of NHS had been added, the mixture was stirred until the total reaction time was as long as 24 hours. The reaction mixture was cooled to room temperature, and after 0.22 ml of concentrated hydrochloric acid was added, the mixture was further stirred for 2 hours. The mixture was placed in a dialysis tube and dialyzed in distilled water for 7 days while the distilled water was changed to new one each day to give an emulsion having a solid content of 7.1 wt.%.

The IR of the above emulsion polymer and the analysis thereof with an X-ray light electron analyzing apparatus (supplied by Shimadzu Corporation, hereinafter referred to as "ESCA") showed that it contained MMA, SSA and Phosmer M. Further, the EA of the polymer showed that the SSA and Phosmer M unit contents were 8.0 and 16.1 mol%, respectively.

Example 13 (EP-2)

While 70 ml of distilled water was temperature-increased to 60° C, an argon gas was bubbled therein for 20 minutes. Under argon atmosphere, 2.00 g (20 mmol) of MMA, 0.74 g (3.5 mmol) of Phosmer M, 0.30 g (1.2 mmol) of SSNa, 30 mg of KPS and 10 mg of NHS were added, and the mixture was vigorously stirred for 3 hours. 10 Minutes after the monomers had been added, the mixture became a bluish white solution.

Further, 1 g (10 mmol) of MMA, 30 mg of KPS and 10 mg of NHS were added, and then 1 g of MMA was added three times at intervals of 20 minutes. After 30 mg of KPS and 10 mg of NHS had been added, the mixture was stirred until the total reaction time was as long as 24 hours. The reaction mixture was cooled to room temperature, and after 0.10 ml of concentrated hydrochloric acid was added, the mixture was further stirred for 2 hours. The mixture was placed in a dialysis tube and dialyzed in distilled water for 7 days while the distilled water was changed to new one each day to give an emulsion having a solid content of 6.2 wt.%.

The IR and ESCA of the above emulsion polymer showed that it contained MMA, SSA and Phosmer M. Further, the EA of the polymer showed that the SSA and Phosmer M unit contents were 5.3 and 2.7 mol%, respectively.

Example 14 (EP-3)

While 70 ml of distilled water was temperature-increased to 60° C, an argon gas was bubbled therein for 20 minutes. Under argon atmosphere, 2.00 g (20 mmol) of MMA, 0.65 g (3.1 mmol) of Phosmer M, 0.17 g (0.7 mmol) of SSNa, 30 mg of KPS and 10 mg of NHS were added, and the mixture was vigorously stirred for 3 hours. 5 Minutes after the monomers had been added, the mixture turned bluish white.

Further, 1 g (10 mmol) of MMA, 30 mg of KPS and 10 mg of NHS were added, and then 1 g of MMA was added three times at intervals of 20 minutes. After 30 mg of KPS and 10 mg of NHS had been added, the mixture was stirred until the total reaction time was as long as 24 hours. The reaction mixture was cooled to room temperature, and after 0.06 ml of concentrated hydrochloric acid was added, the mixture was further stirred for 2 hours. The mixture was placed in a dialysis tube and dialyzed in distilled water for 7 days while the distilled water was changed to new one each day to give an emulsion having a solid content of 6.5 wt.%.

The IR and ESCA of the above emulsion polymer showed that it contained MMA, SSA and Phosmer M. Further, the EA of the polymer showed that the SSA and Phosmer M unit contents were 2.5 and 7.7 mol%, respectively.

Comparative Example 1

All the monomers used in Example 1 were added at one time. That is, 25 ml of distilled water was temperature-increased to 60° C, and a nitrogen gas was bubbled therein for 1 hour. Under nitrogen atmosphere, 5.0 g of MMA,

1.37 g of SSNa, 100 mg of potassium persulfate and 30 mg of sodium sulfite were added at once. In this case, a large amount of aggregates were formed, and no emulsion was obtained.

Comparative Example 2

The emulsion polymerization was carried out in the same manner as in Example 9 except that AA was replaced with sodium acrylate (hereinafter referred to as "ANa"). That is, while 60 ml of distilled water was temperature-increased to 60° C, an argon gas was bubbled therein for 20 minutes. Under argon atmosphere, 2.00 g (20 mmol) of MMA, 0.28 g (3 mmol) of ANa, 0.61 g (2.5 mmol) of SSNa, 30 mg of KPS and 10 mg of NHS were added, and the mixture was vigorously stirred at 60° C. After 20 minutes, a large amount of aggregates were formed, and no emulsion was obtained.

Comparative Example 3

The same emulsion polymerization as that of Example 9 was carried out in distilled water. That is, while 60 ml of distilled water was temperature-increased to 60° C, an argon gas was bubbled therein for 20 minutes. Under argon atmosphere, 2.00 g (20 mmol) of MMA, 0.24 g (3 mmol) of AA, 0.61 g (2.5 mmol) of SSNa, 30 mg of KPS and 10 mg of NHS were added, and the mixture was vigorously stirred at 60° C. Since the formation of a large amount of aggregates was observed after 15 minutes, the reaction was discontinued.

Comparative Example 4 (EMMA)

While 50 ml of distilled water was temperature-increased to 60° C, an argon gas was bubbled therein for 20 minutes. Then, 250 mg of sodium laurylsulfate was added, and the mixture was fully stirred. Thereafter. 10 g (0.1 mol) of MMA, 100 mg of KPS and 10 mg of NHS were added, and the monomers were polymerized for 5 hours. The resultant product was cooled to room temperature, placed in a dialysis tube and dialyzed in distilled water for 7 days while the distilled water was changed to new one each day to give an emulsion having a solid content of 15.7 wt.%.

Examples 15 - 24

Fresh evulsion bovine teeth were polished in water with a water-resistant emery paper (#180) to expose enamels or dentins. Adhesion surfaces were defined by masking tapes having a hole whose diameter was 5.4 mm. Each of the polymer emulsions (E-1 to E-3 and E-5 to E-7) obtained in Examples 1 to 3 and 5 to 7 was adjusted to a solid content of 5 wt.%, and then applied to the adhesion surface in an amount of 3 µl. The applied emulsions were allowed to stand for 1 minutes, and lightly blown by compressed air to form films. MMA was softly applied to the resultant adhesion surfaces twice with a dental brush. Then, acryl rods having a diameter of 6 mm were planted thereon with an MMA-tri-n-butylborane (TBB) resin (X) whose TBB was a polymerization catalyst, and allowed to stand at room temperature for 1 hour to cure the resin. The teeth with the acryl rods planted thereon were immersed in water at 37° C for 24 hours and measured for adhesion strength by subjecting them to a tensile test using an autograph (supplied by Shimadzu Corporation) with a cross head speed of 2 mm/min.

The above procedures after the film formation were repeated using a spontaneous polymerization resin (Y) containing BPO-p-toluidine as a catalyst (Methafast, supplied by Sun Medical Co., Ltd), to measure adhesion strength in the same manner as above.

Further, films were formed in the same manner as above, and a bonding agent (Z) containing 0.5 wt.% of d,l-camphorquinone, 0.5 wt.% of diethylaminobenzoic acid and 99.0 wt.% of triethylene glycol dimethacrylate was applied in an amount of 10 µl and exposed to light from a visible light irradiator (Translux, supplied by Kulzer) for 20 seconds. Then, acryl rings having an internal diameter of 6 mm and a height of 1 mm were placed thereon, and Photobright US (supplied by Kuraray Co., Ltd.) was filled within the ring-formed circles and further exposed to light for 1 minute. Acryl rods were planted with Methafast, and measured for adhesion strength in the same manner as above. Table 1 shows the results.

Table 1

| Example | Emulsion (concentration of 5 wt.%) Code | Dental resin* | Bovine teeth Enamel(EN) /Dentin(DE) | Adhesion strength (MPa) |
|---|---|---|---|---|
| 15 | E-1 | X | EN | 3.1 |
| 16 | E-2 | X | EN | 1.1 |
| 17 | E-3 | X | EN | 1.8 |
| 18 | E-5 | X | EN | 2.3 |
| 19 | E-7 | X | EN | 8.2 |
| 20 | E-7 | X | DE | 2.6 |
| 21 | E-7 | Y | EN | 2.5 |
| 22 | E-7 | Z | EN | 4.0 |
| 23 | E-7 | Z | DE | 1.0 |
| 24 | E-6 | X | EN | 2.3 |
| Blank | No adhesive film | X | EN | 1.0 |
| | No adhesive film | X | DE | 0 |
| | No adhesive film | X | EN | 0 |
| | No adhesive film | Y | DE | 0 |
| | No adhesive film | Y | EN | 0.7 |

* Notes)
X: MMA-TBB resin
Y: MMA-BPO-DMPT resin
X: 3G-CQ-DEABA resin

Examples 25 - 30

Emulsions obtained in Examples (EA-1 to EA-3 and EP-1 to EP-3) were adjusted to a solid content of 5 wt.%, and measured for adhesion strength to enamel in the presence of an MMA/TBB resin (X) in the same manner as above. Table 2 shows the results.

Table 2

| Example | Emulsion (concentration of 5 wt.%) Code | Dental resin* | Bovine teeth Enamel (EN) /Dentin (DE) | Adhesion strength (MPa) |
|---|---|---|---|---|
| 25 | EA-1 | X | EN | 3.0 |
| 26 | EA-2 | X | EN | 2.4 |
| 27 | EA-3 | X | EN | 2.4 |
| 28 | EP-1 | X | EN | 3.4 |
| 29 | EP-2 | X | EN | 2.5 |
| 30 | EP-3 | X | EN | 3.4 |

* Note:
X: MMA-TBB resin

Comparative Examples 5 - 8

Fresh evulsion bovine teeth were polished in water with water-resistant emery paper up to #600. The tooth surface areas were defined by masking tapes having a hole whose diameter was 5.4 mm. Further, acryl rings having an internal diameter of 6 mm and a height of 1 mm were placed thereon, and a commercially available polymer cement was applied within the ring-formed circles and cured. Then, an MMA-TBB resin was filled therein, and the polymer cement was measured for adhesion strength in the same manner as above. The polymer cement was prepared from Hybond Glass Ionomer (HYG, supplied by Shofusha) or Ketac cement (KC, supplied by ESPE) in accordance with the methods specified by each supplier. Table 3 shows the results.

Comparative Examples 9 - 10

The tensile test was carried out by using an MMA/p-styrenesulfonic acid copolymer disclosed in JP-A-62033109,
i.e., a linear MMA-p-styrenesulfonic acid polymer obtained by reacting MMA with SSNa in a water/ethanol-containing solvent and post-treating the resultant reaction product. That is, the copolymer having an MMA content of 70 mol % (MS-7) and the copolymer having an MMA content of 90 mol % (MS-9) were prepared and measured for adhesion strength using a MMA-TBB resin (X) in the same manner as above. Table 3 shows the results.

Table 3

| Comp. Example | Polymer cement and MS | Dental resin* | Bovine teeth Enamel(EN) /Dentin(DE) | Adhesion strength (MPa) |
|---|---|---|---|---|
| 5 | HYG | X | EN | 0.1 |
| 6 | KC | X | EN | 0.3 |
| 7 | HYG | X | DE | 0 |
| 8 | KC | X | DE | 0.2 |
| 9 | MS-7 | X | DE | 0 |
| 10 | MS-9 | X | DE | 0.3 |

* Note:
X: MMA-TBB resin

Example 31

The latex (E-7) having a concentration of 0.05 wt.% was applied to an enamel and dentin, allowed to stand for 1 minute and dried with air. The resultant film was washed with water for 30 minutes, dried and observed through a scanning electron microscope (JMS-5400, JEOL) at an acceleration voltage of 30 KV to show that the film remained on the surfaces, i.e., that the film did not come off when washed with water, since it bonded to the adherends. An adhesion strength to the enamel was 2.0 MPa when MMA-TBB was used as a bonding agent.

Comparative Example 11

A glass surface used as an adhesion surface under the same conditions as those in Example 19 was observed to show no residual film, i.e., that the film completely came off when washed with water.

Examples 32 - 37

The surfaces of synthetic hydroxyapatite having a diameter of 3 mm (supplied by Pentax, hereinafter referred to as "HAP") were fully cleaned. The emulsions obtained in Examples (EA-1 - EA-3 and EP-1 - EP-3) were respectively adjusted to a solid content of 5 wt.%, and applied to HAP surfaces in an amount of 1μl. The applied emulsions were allowed to stand for 1 minute, and lightly blown by compressed air to form films. The films were washed with water for 1 minute and dried. Each film was evaluated for a residual ratio by examining C/Ca before the latices were applied and C/Ca after the films were washed with water by means of an X-ray light electron analyzing apparatus ESCA 500 supplied by Shimadzu Corporation, and calculated a ratio ($C_1$) of C/Ca's.

Table 4 shows the results.

Comparative Example 12

Example 32 was repeated except that the emulsion was replaced with EMMA obtained in Comparative Example 4 and C/Ca ratio of the resultant film was calculated. Table 4 shows the results.

Table 4

| Example | Emulsion (concentration of 5 wt.%) Code | $C_1$* |
|---|---|---|
| 32 | EA-1 | 7.6 |
| 33 | EA-2 | 4.7 |
| 34 | EA-3 | 5.0 |
| 35 | EP-1 | 4.0 |
| 36 | EP-2 | 4.2 |
| 37 | EP-3 | 5.4 |
| Comparative Example 12 | EMMA | 0.8 |

* Note

$$C_1 = \frac{\text{C/Ca (film washed with water)}}{\text{C/Ca (HAP surface)}}$$

Examples 38 - 43

While water was poured, fresh evulsion bovine teeth were polished with water-resistant emery paper (#180) to expose enamels. The emulsions obtained in Examples (EA-1 to EA-3 and EP-1 to EP-3) were adjusted to a solid content of 5 wt.%, and applied to adhesion surfaces in the amount of 1µl. The applied emulsions were allowed to stand for 1 minute and, lightly blown with compressed air to form films. The films were washed with water for 1 minute, dried and observed through a scanning electron microscope (JMS-5400, JEOL) at an acceleration voltage of 10 KV to show that all the films remained and that no film came off.

Example 44

The E-7 emulsion was adjusted to a solid content of 5 wt.%, and applied to sixteen hypersensitive volunteer patients after approval by them. That is, their diseased parts were cleaned, and the emulsion was applied thereto with tampons, allowed to stand and dried with air. Fifteen patients out of the sixteen had a good effect and had no relapse for at least 6 months.

Example 45

(1) Calcium chloride was added to E-7 emulsion having a solid content of 5 wt.% such that the amount of the calcium chloride based on the sulfonic acid group of the E-7 emulsion was 3 equivalent weights. The mixture was shaken for 30 seconds and subjected to centrifugal separation at 1,600 rpm for 10 minutes. The supernatant showed no MS emulsion, and all the contents coagulated.
(2) The above procedures (1) were repeated except that the 3 equivalent weight calcium chloride was replaced with

a 1 equivalent weight aluminum chloride. The supernatant showed no MS emulsion, and all the contents coagulated.

(3) E-7 emulsion having a solid content of 5 wt.% was subjected to centrifugal separation at 1.600 rpm for 10 seconds to show no coagulation.

Example 46

The surfaces of synthetic hydroxyapatite pellets having a diameter of 10 mm and a diameter of 4 mm were cleaned under water current with water-resistant emery paper #600. At room temperature, E-6 emulsion having a solid content of 8 wt.% was applied to the synthetic hydroxyapatite pellet having a diameter of 10 mm, and immediately thereafter, the synthetic hydroxyapatite pellet having a diameter of 4 mm was bonded thereto. The pellets were allowed to stand at room temperature for 12 hours as they were, and then a rod was planted on the synthetic hydroxyapatite pellet having a diameter of 4 mm in the presence of an instantaneous adhesive. Then, the pellets were subjected to a tensile test with an autograph at a cross head speed of 2 mm/min. to show an adhesion strength of 24 $kgf/cm^2$. It is seen that hydroxyapatite pellets can be bonded to each other in the presence of the emulsion of the present invention.

For comparison, the above procedures were repeated except that the E-6 emulsion was replaced with EMMA (Comparative Example 4). As a result, the adhesion strength was 0 $kgf/cm^2$, and it is seen that hydroxyapatite pellets cannot be bonded to each other in the presence of EMMA.

## Claims

1. Use of a composition containing an emulsion of a polymer comprising (a) a recurring unit derived from a (meth)acrylate and (b) a recurring unit derived from a vinyl compound containing a $-SO_3R$ group in which R is hydrogen, an alkali metal or an ammonium ion as a dental adhesive.

2. Use of an emulsion of a polymer as defined in claim 1 to form an anti-hypersensitivity film on dentin.

3. Use according to claim 1 or claim 2, wherein the polymer further comprises (c) a recurring unit derived from a vinyl compound containing a $-COOR_4$ or $-OPO(OR_5)_2$ group in which $R_4$ and $R_5$ are each hydrogen, an alkali metal or an ammonium ion.

4. Use according to claim 1 or claim 2, wherein the polymer is derived from a mixture comprising the (meth)acrylate and the vinyl compound containing a $-SO_3R$ group in a (meth)acrylate:vinyl compound molar ratio of from 99:1 to 50:50.

5. Use according to claim 3, wherein the polymer is derived from a mixture comprising the (meth)acrylate, the vinyl compound containing a $-SO_3R$ group and the vinyl compound containing a $-COOR_4$ or $-OPO(OR_5)_2$ group in a molar ratio of (meth)acrylate:total of the two compounds of from 99:1 to 50:50.

6. Use according to claim 5, wherein the molar ratio of the vinyl compound containing a $-SO_3R$ group to the vinyl compound containing a $COOR_4$ or $-OPO(OR_5)2$ group is from 99:1 to 1:99.

7. Use according to any one of claims 1 to 6, wherein the recurring unit derived from a (meth)acrylate has the formula (A)

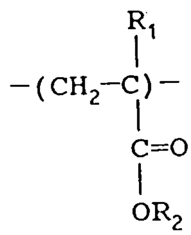

wherein $R_1$ is hydrogen or $C_1$ to $C_5$ alkyl and $R_2$ is $C_1$ to $C_5$ alkyl, and the recurring unit derived from a vinyl compound containing a $-SO_3R$ group has the formula (B)

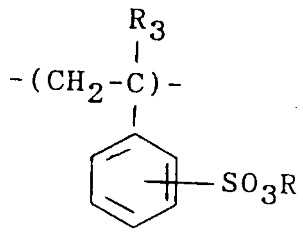

wherein $R_3$ is hydrogen or $C_1$ to $C_5$ alkyl and R is hydrogen, an alkali metal or an ammonium ion.

**8.** Use according to any one of claims 1 to 7 wherein the polymer is obtainable by emulsion polymerisation.

**9.** Use according to any one of claims 1 to 7 wherein the emulsion is obtainable by emulsion-polymerizing a (meth)acrylate in the presence of an emulsion as defined in any one of claims 1 to 6 as an emulsifying agent.

**10.** Use according to claim 9, wherein the polymer obtainable by emulsion polymerising a (meth)acrylate comprises a recurring unit of formula (A) as defined in claim 7.

**11.** Use according to any one of claims 1 to 10 wherein the composition or film further comprises a low molecular weight acid.

**12.** Use according to claim 11, wherein the low molecular weight acid is citric acid, oxalic acid, maleic acid, phosphoric acid or EDTA.

**13.** Use according to any one of claims 1 or 3 to 12, wherein the dental adhesive composition is a dental primer.

**Patentansprüche**

**1.** Verwendung einer Masse, die eine Emulsion aus einem Polymer enthält, umfassend (a) eine Wiederholungseinheit, abgeleitet von einem (Meth)acrylat, und (b) eine Wiederholungseinheit, abgeleitet von einer Vinylverbindung, die eine -$SO_3$R-Gruppe enthält, in welcher R Wasserstoff ein Alkalimetall- oder ein Ammoniumion ist, als Dentalklebstoff.

**2.** Verwendung einer Emulsion eines Polymers, wie in Anspruch 1 definiert, zur Bildung eines Anti-Überempfindlichkeitsfilms auf Dentin.

**3.** Verwendung gemäß Anspruch 1 oder Anspruch 2, wobei das Polymer weiterhin (c) eine Wiederholungseinheit, abgeleitet von einer Vinylverbindung, enthaltend eine -$COOR_4$- oder -$OPO(OR_5)_2$-Gruppe, in welcher $R_4$ und $R_5$ jeweils Wasserstoff, ein Alkalimetall- oder ein Ammoniumion sind, umfaßt.

**4.** Verwendung gemäß Anspruch 1 oder Anspruch 2, wobei das Polymer abgeleitet ist von einer Mischung, umfassend das (Meth)acrylat und die Vinylverbindung, enthaltend eine - $SO_3$R-Gruppe, in einem (Meth)acrylat : Vinylverbindungs-Molverhältnis von 99 : 1 bis 50 : 50.

**5.** Verwendung gemäß Anspruch 3, wobei das Polymer abgeleitet ist von einer Mischung, umfassend das (Meth)acrylat, die Vinylverbindung, enthaltend eine -$SO_3$R-Gruppe, und die Vinylverbindung, enthaltend eine -$COOR_4$- oder -$OPO(OR_5)_2$-Gruppe in einem Molverhältnis von (Meth)acrylat : Gesamtheit der zwei Verbindungen von 99 : 1 bis 50 : 50.

**6.** Verwendung gemäß Anspruch 5, wobei das Molverhältnis der Vinylverbindung, die eine -$SO_3$R-Verbindung enthält, zu der Vinylverbindung, die eine $COOR_4$- oder $OPO(OR_5)_2$-Gruppe enthält, 99 : 1 bis 1 : 99 beträgt.

**7.** Verwendung gemäß mindestens einem der Ansprüche 1 bis 6, wobei die von einem (Meth)acrylat abgeleitete Wiederholungseinheit die Formel (A) besitzt:

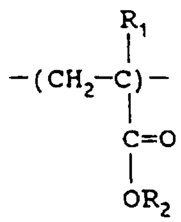

worin $R_1$ Wasserstoff oder $C_1$- bis $C_5$-Alkyl ist und $R_2$ $C_1$- bis $C_5$-Alkyl ist und die Wiederholungseinheit, abgeleitet von einer Vinylverbindung, die eine $-SO_3R$-Gruppe enthält, die Formel (B) besitzt:

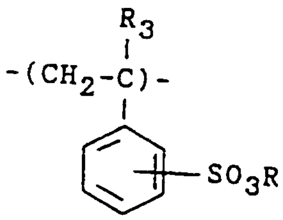

worin $R_3$ Wasserstoff oder $C_1$- bis $C_5$-Alkyl ist und R ein Wasserstoff-, ein Alkalimetall- oder ein Ammoniumion ist.

**8.** Verwendung gemäß mindestens einem der Ansprüche 1 bis 7, wobei das Polymer durch Emulsionspolymerisation erhältlich ist.

**9.** Verwendung gemäß mindestens einem der Ansprüche 1 bis 7, wobei die Emulsion durch Emulsionspolymerisierung eines (Meth)acrylats in Gegenwart einer Emulsion erhältlich ist, wie in mindestens einem der Ansprüche 1 bis 6 definiert, als ein Emulgiermittel.

**10.** Verwendung gemäß Anspruch 9, wobei das durch Emulsionspolymerisierung eines (Meth)acrylats erhältliche Polymer eine Wiederholungseinheit der Formel (A), wie in Anspruch 7 definiert, umfaßt.

**11.** Verwendung gemäß mindestens einem der Ansprüche 1 bis 10, wobei die Zusammensetzung oder der Film weiterhin eine Säure mit niedrigem Molekulargewicht umfaßt.

**12.** Verwendung gemäß Anspruch 11, wobei die Säure mit niedrigem Molekulargewicht Citronensäure, Oxalsäure, Maleinsäure, Phosphorsäure oder EDTA ist.

**13.** Verwendung gemäß mindestens einem der Ansprüche 1 oder 3 bis 12, wobei die dentale Klebemasse eine dentale Grundierung ist.

**Revendications**

**1.** Utilisation comme adhésif dentaire d'une composition contenant une émulsion d'un polymère comprenant (a) des motifs issus d'un acrylate ou méthacrylate et (b) des motifs issus d'un composé vinylique contenant un groupe $-SO_3R$ dans lequel R désigne un atone d'hydrogène, un atome de métal alcalin ou un ion ammonium.

**2.** Utilisation d'une émulsion d'un polymère telle que définie dans la revendication 1 pour former sur la dentine un film contre l'hypersensibilité.

**3.** Utilisation selon la revendication 1 ou 2, pour laquelle le polymère comprend en outre (c) des motifs issus d'un composé vinylique contenant un groupe $-COOR_4$ ou $-OPO(OR_5)_2$ dans lequel $R_4$ et $R_5$ représentent chacun un atome d'hydrogène, un atome de métal alcalin ou un ion ammonium.

**4.** Utilisation selon la revendication 1 ou 2, pour laquelle le polymère est issu d'un mélange comprenant l'acrylate ou méthacrylate et le composé vinylique à groupe $-SO_3R$ selon un rapport molaire acrylate ou méthacrylate:composé

vinylique égal à 99:1 à 50:50.

**5.** Utilisation selon la revendication 3, pour laquelle le polymère est issu d'un mélange comprenant l'acrylate ou méthacrylate, le composé vinylique à groupe $-SO_3R$ et le composé vinylique à groupe $-COOR_4$ ou $-OPO(OR_5)_2$ selon un rapport molaire acrylate ou méthacrylate:total des deux composés égal à 99:1 à 50:50.

**6.** Utilisation selon la revendication 5, pour laquelle le rapport molaire du composé vinylique à groupe $-SO_3R$ au composé vinylique à groupe $-COOR_4$ ou $-OPO(OR_5)_2$ est égal à 99:1 à 1:99.

**7.** Utilisation selon l'une quelconque des revendications 1 à 6, pour laquelle les motifs issus d'un acrylate ou méthacrylate répondent à la formule (A)

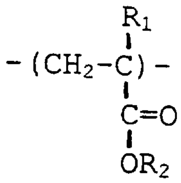

dans laquelle $R_1$ représente un atome d'hydrogène ou un groupe alkyle en $C_1$ à $C_5$ et $R_2$ représente un groupe alkyle en $C_1$ à $C_5$, et les motifs issus d'un composé vinylique contenant un groupe $-SO_3R$ répondent à la formule (B)

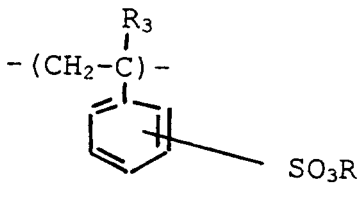

dans laquelle $R_3$ représente un atome d'hydrogène ou un groupe alkyle en $C_1$ à $C_5$ et R représente un atome d'hydrogène, un atome de métal alcalin ou un ion ammonium.

**8.** Utilisation selon l'une quelconque des revendications 1 à 7, pour laquelle le polymère peut être obtenu par polymérisation en émulsion.

**9.** Utilisation selon l'une quelconque des revendications 1 à 7, pour laquelle l'émulsion peut être obtenue par polymérisation en émulsion d'un acrylate ou méthacrylate en présence d'une émulsion telle que définie dans l'une quelconque des revendications 1 à 6, en tant qu'émulsufiant.

**10.** Utilisation selon la revendication 9, pour laquelle le polymère préparable par polymérisation en émulsion d'un acrylate ou méthacrylate comprend des motifs de formule (A) tels que définis dans la revendication 7.

**11.** Utilisation selon l'une quelconque des revendications 1 à 10, pour laquelle la composition ou le film comprend en outre un acide de faible masse moléculaire.

**12.** Utilisation selon la revendication 11, pour laquelle l'acide de faible masse moléculaire est l'acide citrique, l'acide oxalique, l'acide maléique, l'acide phosphorique ou l'EDTA.

**13.** Utilisation selon l'une quelconque des revendications 1 et 3 à 12, dans laquelle la composition pour adhésif dentaire est une couche d'apprêt dentaire.